(19) Europäisches Patentamt / European Patent Office / Office européen des brevets

(11) **EP 3 245 511 B1**

(12) **FASCICULE DE BREVET EUROPEEN**

(45) Date de publication et mention de la délivrance du brevet:
**22.06.2022 Bulletin 2022/25**

(21) Numéro de dépôt: **16700444.9**

(22) Date de dépôt: **13.01.2016**

(51) Classification Internationale des Brevets (IPC):
**G01N 33/28** *(2006.01)*    **G01N 33/30** *(2006.01)*
**G01N 21/78** *(2006.01)*    **G01N 33/20** *(2019.01)*

(52) Classification Coopérative des Brevets (CPC):
**G01N 21/78; G01N 33/2835; G01N 33/2858; G01N 33/2888; G01N 33/30;** G01N 33/20

(86) Numéro de dépôt international:
**PCT/EP2016/050523**

(87) Numéro de publication internationale:
**WO 2016/113281 (21.07.2016 Gazette 2016/29)**

(54) **MÉTHODE ET KIT POUR LE DOSAGE DES IONS FER DANS LES COMPOSITIONS LUBRIFIANTES**

VERFAHREN UND KIT ZUM DOSIEREN VON EISENIONEN IN SCHMIERMITTELZUSAMMENSETZUNGEN

METHOD AND KIT FOR DOSING IRON IONS IN LUBRICATING COMPOSITIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.01.2015 FR 1550285**

(43) Date de publication de la demande:
**22.11.2017 Bulletin 2017/47**

(73) Titulaire: **Total Marketing Services**
**92800 Puteaux (FR)**

(72) Inventeurs:
• **SIGNOL, Catherine**
  **92700 Colombes (FR)**
• **DARGENT, Luc**
  **33200 Bordeaux (FR)**
• **MAIROT, Grégory**
  **33370 Pompignac (FR)**
• **DEBUISSIER, Thomas**
  **33370 Salleboeuf (FR)**
• **DEMAILLE, Camille**
  **33800 Bordeaux (FR)**
• **BARON, Christian**
  **87100 Limoges (FR)**

(74) Mandataire: **Lavoix**
  **62, rue de Bonnel**
  **69448 Lyon Cedex 03 (FR)**

(56) Documents cités:
**US-A- 4 238 197    US-A1- 2006 270 050**

• **SENEE KRUANETR ET AL: "A Simple Flow Injection Spectrophotometric Determination of Iron Using Nitroso-R salt as complexing agent", J. FLOW INJECTION ANAL, vol. 24, 1 janvier 2007 (2007-01-01), pages 114-118, XP055248165,**
• **SALVADOR A ET AL: "Determination of the total iron content of used lubricating oils by atomic-absorption with use of emulsions", TALANTA, ELSEVIER, AMSTERDAM, NL, vol. 30, no. 12, 1 décembre 1983 (1983-12-01), pages 986-988, XP026596345, ISSN: 0039-9140, DOI: 10.1016/0039-9140(83)80230-6 [extrait le 1983-12-01]**

**Description**

La présente demande concerne une méthode de dosage du fer corrosif, c'est-à-dire des ions ferriques et ferreux, dans les compositions lubrifiantes usagées, plus particulièrement dans les compositions lubrifiantes usagées de moteur, plus particulièrement de moteur marin. La présente demande concerne également un kit pour la mise en œuvre de cette méthode.

Les moteurs, notamment les moteurs marins, subissent des contraintes importantes lors de leur fonctionnement, notamment contrainte de friction, et sont soumis à la corrosion, notamment, par exemple dans le cas des moteurs marins, du fait de la présence de soufre dans le fuel qui est transformé en acide sulfurique lors de la combustion. Des compositions lubrifiantes sont généralement utilisées pour diminuer ces contraintes.

De par ces phénomènes, notamment de friction et de corrosion, des particules ou contaminants métalliques, notamment fer particulaire, ions ferriques et ions ferreux, sont libérées dans ces compositions lubrifiantes. Ces contaminants métalliques, s'ils sont présents en quantité trop importante, peuvent entraîner une modification des propriétés desdites compositions lubrifiantes et donc de leurs performances et être dommageables pour le moteur (notamment diminution de la durée de vie). Il y a donc un intérêt à doser ces contaminants métalliques dans les compositions lubrifiantes pour déterminer l'état de dégradation de ces compositions et ainsi le moment optimal du changement de celles-ci. De plus, le dosage de ces contaminants métalliques permet également de déterminer l'importance de ces phénomènes, notamment de friction et de corrosion, et de prendre des décisions pour l'entretien et la préservation du matériel lubrifié, notamment l'ajustement de la quantité de composition lubrifiante à introduire.

Des laboratoires proposent d'effectuer des analyses d'échantillons de compositions lubrifiantes usagées pour déterminer les quantités de contaminants métalliques. Cependant, cela nécessite, l'envoi des échantillons, un temps de mise en œuvre important et par conséquent des actions retardées.

Il existe donc un intérêt à fournir une méthode de dosage du fer, notamment des ions ferriques et ferreux issus de la corrosion, dans les compositions lubrifiantes usagées et un kit adapté à la mise en œuvre de cette méthode, qui puissent être mis en œuvre sur place, qui soient rapides et fiables.

Il existe des tests colorimétriques, dont la détermination d'une gamme de concentration en fer se fait de façon visuelle par comparaison avec des échantillons de référence. Cependant, ces méthodes sont peu fiables.

Il est connu également de WO2006127098 et US4238197 une méthode pour analyser la quantité de fer contenue dans une composition lubrifiante usagée. Cependant, cette méthode permet le dosage du fer total contenu dans ladite composition lubrifiante, c'est-à-dire tant le fer particulaire issu des phénomènes de friction, que les ions ferriques et ferreux issus de la corrosion. Il n'est donc pas possible, par cette méthode, de déterminer le réel impact de chacun de ces deux phénomènes pris séparément et, pour les opérateurs, d'agir en conséquence.

Un des objectifs de la présente invention est par conséquent de fournir une méthode de dosage « sur place » des ions fer, ions ferriques et ferreux, notamment issus de la corrosion des pièces métalliques des moteurs, dans les compositions lubrifiantes usagées, notamment récupérées en bas des cylindres de moteur.

Un autre objectif de la présente invention est de fournir une telle méthode pour laquelle la préparation de l'échantillon pour le dosage est simple et rapide.

Un autre objectif encore de la présente invention est de fournir une telle méthode qui soit de mise en œuvre rapide, de préférence qui nécessite moins de 10 minutes, qui soit fiable et reproductible.

Un autre objectif de la présente invention est de fournir une méthode permettant une précision de mesure de plus ou moins 10 ppm et permettant une mesure dans la gamme 0 à 900 ppm.

Un autre objectif encore de la présente invention est de fournir un kit permettant de mettre en œuvre ladite méthode, qui soit d'utilisation simple et rapide.

D'autres objectifs encore apparaîtront à la lecture de la description de l'invention qui suit.

Tous ces objectifs sont remplis par la présente invention qui propose une méthode de dosage « sur place » des ions fer, notamment des ions ferriques et ferreux, dans les compositions lubrifiantes, notamment les compositions lubrifiantes usagées, par exemple récupérées en bas des cylindres de moteur, par mesure photochimique après réaction des ions fer avec un agent complexant des ions fer, la réaction de complexation amenant un changement de couleur pouvant être quantifié par spectrophotométrie.

La présente invention concerne une méthode de dosage des ions fer selon la revendication 1.

Il doit être compris de l'étape a) de la revendication 1 que c'est l'échantillon prélevé qui est introduit dans un premier récipient.

La composition lubrifiante à analyser est de préférence une composition lubrifiante usagée de moteur, de préférence une composition lubrifiante de moteur marin, par exemple de moteur de navire ou de centrale hydroélectrique, etc. De préférence, la composition lubrifiante est une composition lubrifiante pour moteur marin 2 temps. De préférence, la composition lubrifiante à analyser est une composition lubrifiante usagée récupérée en bas des cylindres de moteur.

La composition lubrifiante dont il est question dans la présente invention comprend au moins une huile de base lubrifiante. En général, les huiles de base lubrifiantes peuvent être des huiles d'origine minérales, synthétiques ou végétales ainsi que leurs mélanges. Les huiles minérales ou synthétiques généralement utilisées appartiennent à l'un des groupes I à V selon les classes définies dans la classification API (ou leurs équivalents selon la classification ATIEL) telle que résumée ci-dessous. La classification API est définie dans American Petroleum Institute 1509 "Engine oil Licensing and Certification System" 17ieme édition, Septembre 2012. La classification ATIEL est définie dans "The ATIEL Code of Practice", numéro 18, Novembre 2012.

|  | Teneur en saturés | Teneur en soufre | Indice de viscosité |
|---|---|---|---|
| Groupe I Huiles minérales | < 90% | > 0,03 % | $80 \leq VI < 120$ |
| Groupe II Huiles hydrocraquées | $\geq 90\%$ | $\leq 0,03$ % | $80 \leq VI < 120$ |
| Groupe III Huiles hydrocraquées ou hydroisomérisées | $\geq 90$ % | $\leq 0,03$ % | $\geq 120$ |
| Groupe IV | PAO (Poly alpha olefins) | | |
| Groupe V | Esters et autres bases non incluses dans bases groupes I à IV | | |

Les huiles minérales de Groupe I peuvent être obtenues par distillation de bruts naphténiques ou paraffiniques sélectionnés puis purification des distillats obtenus par des procédés tels que extraction par solvant, déparaffinage au solvant ou catalytique, hydrotraitement ou hydrogénation. Les huiles des Groupes II et III sont obtenues par des procédés de purification plus poussés, par exemple une combinaison de traitement choisi parmi l'hydrotraitement, l'hydrocraquage, l'hydrogénation et le déparaffinage catalytique. Des exemples d'huiles de base synthétiques de Groupe IV et V incluent les polyisobutènes, les alkylbenzènes et les poly-alphas oléfines telles que les polybutènes ou encore les esters.

Dans les compositions lubrifiantes, les huiles de base lubrifiantes peuvent être utilisées seules ou en mélange. Par exemple, une huile minérale peut être combinée avec une huile synthétique.

Les huiles cylindres pour moteurs marins deux temps sont généralement caractérisées par un grade viscosimétrique SAE-40 à SAE-60, généralement SAE-50 équivalent à une viscosité cinématique à 100°C comprise entre 16,3 et 21,9 mm$^2$/s mesurée selon la norme ASTM D445. Les huiles de grade SAE-40 ont une viscosité cinématique à 100°C comprise entre $12,5 \times 10^{-6}$ et $16,3 \times 10^{-6}$ m$^2$.s$^{-1}$ (soit 12,5 et 16, 3 cSt, respectivement) mesurée selon la norme ASTM D445. Les huiles de grade SAE-50 ont une viscosité cinématique à 100°C comprise entre $16,3 \times 10^{-6}$ et $21,9 \times 10^{-6}$ m$^2$.s$^{-1}$ (soit 16,3 et 21,9 cSt, respectivement) mesurée selon la norme ASTM D445. Les huiles de grade SAE-60 ont une viscosité cinématique à 100°C comprise entre $21,9 \times 10^{-6}$ et $26,1 \times 10^{-6}$ m$^2$.s$^{-1}$ (soit 21,9 et 26,1 cSt, respectivement) mesurée selon la norme ASTM D445. Les compositions lubrifiantes de l'invention ont, de préférence, une viscosité cinématique mesurée selon la norme ASTM D445 à 100°C allant de $12,5 \times 10^{-6}$ et $26,1 \times 10^{-6}$ m$^2$.s$^{-1}$ (soit 12,5 et 26,1 cSt, respectivement), préférentiellement de $16,3 \times 10^{-6}$ et $21,9 \times 10^{-6}$ m$^2$.s$^{-1}$ (soit 16,3 et 21,9 cSt, respectivement). Pour obtenir de telle viscosité, les compositions lubrifiantes de l'invention peuvent comprendre en outre un ou plusieurs additifs. Typiquement, une formulation classique de composition lubrifiante pour moteurs marins, de préférence deux temps, est de grade SAE-40 à SAE-60, préférentiellement SAE-50 (selon la classification SAE J300) et comprend au moins 40 % en poids d'huile de base lubrifiante d'origine minérale, synthétique ou leurs mélanges, adaptée à l'utilisation pour un moteur marin. Par exemple, une huile de base lubrifiante de groupe I, selon la classification API, peut être utilisée pour la formulation d'un lubrifiant cylindre. Les huiles de base lubrifiantes de groupe I ont un Indice de Viscosité (VI) allant de 80 à 120 ; leur teneur en soufre est supérieure à 0,03 % et leur teneur en composés hydrocarbonés saturés est inférieure à 90 %.

La composition lubrifiante selon l'invention peut comprendre en outre un additif choisi parmi les détergents surbasés ou les détergents neutres. Les détergents sont typiquement des composés anioniques comportant une longue chaîne hydrocarbonée lipophile et une tête hydrophile, le cation associé est typiquement un cation métallique d'un métal alcalin ou alcalino-terreux. Les détergents sont préférentiellement choisis parmi les sels de métaux alcalins ou alcalino-terreux (notamment préférentiellement le calcium, le magnésium, le sodium ou le baryum) d'acides carboxyliques, sulfonates, salicylates, naphténates, ainsi que les sels de phénates. Ces sels métalliques peuvent contenir le métal en quantité approximativement stœchiométrique par rapport au(x) groupement(s) anionique(s) du détergent. Dans ce cas, on parle de détergents non surbasés ou « neutres », bien qu'ils apportent également une certaine basicité. Ces détergents « neutres » ont typiquement un BN (Base Number ou Indice de basicité), mesuré selon ASTM D2896, inférieur à 150 mg KOH/g, ou inférieur à 100 mg KOH/g, ou encore inférieur à 80 mg KOH/g de détergent. Ce type de détergents dits neutres peut contribuer pour partie au BN des compositions lubrifiantes. Sont employés par exemple des détergents neutres de type carboxylates, sulfonates, salicylates, phénates, naphténates de métaux alcalins et alcalino-terreux, par exemple de calcium, sodium, magnésium, baryum. Lorsque le métal est en excès (en quantité supérieure à la quantité stoechiométrique par rapport au(x) groupements(s) anionique(s) du détergent), on a affaire à des détergents dits surbasés. Leur BN est élevé, supérieur à 150 mg KOH/g de détergent, typiquement allant de 200 à 700 mg KOH/g de détergent, préférentiellement de 250 à 450 mg KOH/g de détergent. Le métal en excès apportant le caractère surbasé au détergent se présente sous la forme de sels métalliques insolubles dans l'huile, par exemple carbonate, hydroxyde, oxalate, acétate, glutamate, préférentiellement carbonate. Dans un même détergent surbasé, les métaux de ces sels insolubles peuvent être les mêmes que ceux des détergents solubles dans l'huile ou bien être différents. Ils sont préférentiellement choisis parmi le calcium, le magnésium, le sodium ou le baryum. Les détergents surbasés se présentent ainsi sous forme de micelles composées de sels métalliques insolubles maintenues en suspension dans la composition lubrifiante par les détergents sous forme de sels métalliques solubles dans l'huile. Ces micelles peuvent contenir un ou plusieurs types de sels métalliques insolubles, stabilisés par un ou plusieurs types détergents. Les détergents surbasés comportant un seul type de sel métallique soluble détergent seront généralement nommés d'après la nature de la chaîne hydrophobe de ce dernier détergent. Ainsi, ils seront dits de type phénate, salicylate, sulfonate, naphténate selon que ce détergent est respectivement un phénate, salicylate, sulfonate, ou naphténate. Les détergents surbasés seront dits de type mixte si les micelles comprennent plusieurs types de détergents, différents entre eux par la nature de leur chaîne hydrophobe. Le détergent surbasé et le détergent neutre peuvent être choisis parmi les carboxylates, sulfonates, salicylates, naphténates, phénates, et les détergents mixtes associant au moins deux de ces types de détergents. Le détergent surbasé et le détergent neutre sont notamment des composés à base de métaux choisis parmi le calcium, le magnésium, le sodium ou le baryum, préférentiellement le calcium ou le magnésium. Le détergent surbasé peut être surbasé par des sels insolubles métalliques choisis dans le groupe des carbonates de métaux alcalins et alcalino-terreux, préférentiellement le carbonate de calcium. La composition lubrifiante peut comprendre au moins un détergent surbasé et au moins un détergent neutre tels que définis ci-dessus.

Comme mentionné ci-dessus, dans un mode de réalisation de l'invention, la composition lubrifiante a un BN déterminé selon la norme ASTM D-2896 d'au plus 50, de préférence d'au plus 40, avantageusement d'au plus 30 milligrammes de potasse par gramme de composition lubrifiante, notamment allant de 10 à 30, de préférence de 15 à 30, avantageusement de 15 à 25 milligrammes de potasse par gramme de composition lubrifiante. Dans ce mode de réalisation de l'invention, la composition lubrifiante peut ne pas comprendre de détergents à base de métaux alcalins ou alcalino-terreux surbasés par des sels métalliques de carbonate.

Dans un autre mode de réalisation de l'invention, la composition lubrifiante a un BN déterminé selon la norme ASTM D-2896 d'au moins 50, préférentiellement d'au moins 60, plus préférentiellement d'au plus 70, avantageusement de 70 à 100.

La composition lubrifiante peut également comprendre au moins un autre additif supplémentaire choisi parmi les dispersants, les additifs anti-usure ou tout autre additif fonctionnel. Les dispersants sont des additifs bien connus employés dans la formulation de composition lubrifiante, notamment pour application dans le domaine marin. Leur rôle premier est de maintenir en suspension les particules présentes initialement ou apparaissant dans le lubrifiant au cours de son utilisation dans le moteur. Ils préviennent leur agglomération en jouant sur l'encombrement stérique. Ils peuvent présenter également un effet synergique sur la neutralisation. Les dispersants utilisés comme additifs pour lubrifiant contiennent typiquement un groupement polaire, associé à une chaîne hydrocarbonée relativement longue, contenant généralement de 50 à 400 atomes de carbone. Le groupement polaire contient typiquement au moins un élément azote, oxygène ou phosphore. Les composés dérivés de l'acide succinique sont des dispersants particulièrement utilisés comme additifs de lubrification. On utilise en particulier les succinimides, obtenus par condensation d'anhydrides succiniques et d'amines, les esters succiniques obtenus par condensation d'anhydrides succiniques et d'alcools ou polyols. Ces composés peuvent être ensuite traités par divers composés notamment soufre, oxygène, formaldéhyde, acides carboxyliques et composés contenant du bore ou du zinc pour produire par exemple des succinimides boratées ou des succinimides bloqués au zinc. Les bases de Mannich, obtenues par polycondensation de phénols substitués par des groupements alkyles, de formaldéhyde et d'amines primaires ou secondaires, sont également des composés utilisés comme dispersants dans les lubrifiants. Dans un mode de réalisation de l'invention, la teneur en dispersant peut être supérieure ou égal à 0,1%, de préférence de 0,5 à 2%, avantageusement de 1 à 1,5 % en poids par rapport au poids total de la composition lubrifiante. Les additifs anti-usure protègent les surfaces en frottement par formation d'un film protecteur adsorbé sur ces surfaces. Le plus couramment utilisé est le di thiophosphate de zinc ou DTPZn. On trouve également dans cette catégorie divers composés phosphorés, soufrés, azotés, chlorés et borés. Il existe une grande variété d'additifs anti-usure, mais la catégorie la plus utilisée est celle des additifs phospho soufrés comme les alkylthiophosphates métalliques, en particulier les alkylthiophosphates de zinc, et plus spécifiquement les dialkyldithiophosphates de zinc ou DTPZn. Les composés préférés sont de formule $Zn((SP(S)(OR_1)(OR_2))2$ , ou $R_1$ et $R_2$ sont des groupements alkyl , comportant préférentiellement de 1 à 18 atomes de carbones. Le DTPZn est typiquement présent à des teneurs de l'ordre de 0,1 à 2 % en poids par rapport au poids total de la composition lubrifiante. Les phosphates d'amines, les polysulfures, notamment les oléfines soufrées, sont également des additifs anti-usure employés couramment. On rencontre également usuellement dans les compositions lubrifiantes pour moteur marin des additifs anti-usure et extrême pression de type azotés et soufrés, tels que par exemple les dithiocarbamates métalliques, en particulier dithiocarbamate de molybdène. Les esters du glycérol sont également des additifs anti usure. On peut citer par exemple les mono, di et trioléates, monopalmitates et monomyristates. Dans un mode de réalisation, la teneur en additifs anti-usure va de 0,01 à 6 %, préférentiellement de 0,1 à 4 % en poids par rapport au poids total de la composition lubrifiante.

Les autres additifs fonctionnels peuvent être choisis parmi les agents épaississants, les additifs anti-mousse pour contrer l'effet des détergents, pouvant être par exemple des polymères polaires tels que polyméthylsiloxanes, polyacrylates, les additifs anti-oxydant et/ou anti rouille, par exemple détergents organo-métalliques ou thiadiazoles. Ceux-ci sont connus de l'homme du métier. Ces additifs sont généralement présents à une teneur en poids de 0,1 à 5% par rapport au poids total de la composition lubrifiante.

Dans la méthode de la présente invention, l'étape b) permet d'obtenir un dépôt au fond du premier récipient du fer particulaire éventuellement contenu dans la composition lubrifiante à analyser et résultant de la friction des pièces de moteur.

Le prélèvement de l'étape e) est réalisé sur le surnageant ce qui permet d'effectuer le dosage uniquement des ions ferriques et ferreux et ainsi de déterminer l'importance du seul phénomène de corrosion.

Dans le cadre de la présente invention, on entend par « ion fer » désigner les ions ferriques et les ions ferreux.

Les compositions lubrifiantes sont généralement composées d'huiles lubrifiantes et d'additifs et sont donc visqueuses et généralement colorées. Il n'est ainsi pas aisé de réaliser le dosage colorimétrique des ions fer directement dans la composition lubrifiante. La présente invention propose donc de faire passer les ions fer en phase aqueuse. De façon avantageuse, la première composition réactive (CR1) de la méthode selon la présente invention comprend en solution aqueuse au moins un agent d'extraction des ions ferriques et ferreux de la phase huileuse de la composition lubrifiante vers la phase aqueuse. L'agent d'extraction permet de faire passer l'ensemble des ions ferriques et ferreux de la phase huileuse de la composition lubrifiante vers la phase aqueuse, l'eau provenant notamment de la composition CR1, dans laquelle sera réalisée la complexation avec l'agent complexant et le dosage. Cet agent d'extraction est notamment choisi parmi les agents solubilisant les ions ferriques et ferreux et étant immiscibles à la composition lubrifiante. Parmi ces agents d'extraction on peut notamment citer les acides, de préférence les acides ayant un pKa compris entre -5 et +5, notamment acide sulfurique, acide nitrique, acide hydrochlorhydrique, acide phosphorique, seuls ou en mélange. De préférence, l'acide présente un pKa de -3.

La composition CR1 peut en outre comprendre d'autres constituants, notamment des additifs aidant à la préparation de la composition et permettant avantageusement d'augmenter la rapidité de l'analyse. Parmi ces additifs on peut citer les co-solvants aidant à la solubilisation des composés de la composition réactive CR1, par exemple de type alcool, par exemple éthanol, 1,4-butanediol, de préférence éthanol.

Dans la deuxième composition réactive (CR2) de la présente invention, l'agent de réduction des ions ferriques en ions ferreux est de préférence choisi parmi les agents de réduction suivant : hydroquinone, chlorhydrate d'hydroxylamine, hydrazine, dithionite, seuls ou en mélange. L'agent de réduction permet d'obtenir une solution comprenant uniquement des ions fer sous une seule forme (entité) et par conséquent d'avoir un dosage plus fiable et précis.

La composition CR2 peut en outre comprendre des solutions tampon permettant de conserver le pH sensiblement stable, de préférence entre 2 et 7, plus préférentiellement entre 2 et 3. Parmi les solutions tampon on peut citer des solutions de base ou d'acide ou leur mélange, par exemple des solutions, notamment concentrées d'un acide préférentiellement faible et de sa base conjuguée, des sels, par exemple des sels de sodium tel que l'acétate de sodium, ou leur mélange. De préférence, la solution tampon est de préférence choisie parmi les solutions d'acétate de sodium et d'acide, notamment acide acétique, les solutions de glycine et d'acide chlorhydrique, les solutions d'acide éthanoïque et éthanoate de sodium ou les solutions d'acide citrique et phosphate de sodium.

De préférence, la première composition réactive selon la présente invention comprend de préférence de 1 à 10 % en poids d'agent d'extraction. De préférence, la deuxième composition réactive selon la présente invention comprend de 1 à 15% en poids d'agent de réduction.

De façon particulièrement avantageuse, les première et deuxième compositions réactives peuvent être mélangées en une unique composition réactive aqueuse (CR1') comprenant alors au moins un agent d'extraction des ions ferriques et ferreux de la phase huileuse vers la phase aqueuse, l'eau provenant notamment de la composition CR1', et au moins un agent de réduction des ions ferriques (Fe$^{3+}$) en ions ferreux (Fe$^{2+}$). De préférence, la composition réactive CR1' comprend de 1 à 15 % en poids d'agent de réduction et de 1 à 10% en poids d'agent d'extraction. Cette composition réactive (CR1') peut en outre comprendre d'autres constituants, notamment des additifs aidant à la préparation de la composition et permettant avantageusement d'augmenter la rapidité de l'analyse. Parmi ces additifs on peut citer les co-solvants aidant à la solubilisation des composés de la composition réactive CR1', par exemple de type alcool, par exemple éthanol ou 1,4-butanediol, de préférence éthanol et/ou des solutions tampon permettant de conserver le pH sensiblement stable, de préférence entre 2 et 7, plus préférentiellement entre 2 et 3, parmi les solutions tampon on peut citer des solutions de base ou d'acide ou leur mélange, par exemple des solutions, notamment concentrées d'un acide faible et de sa base conjuguée, des sels, par exemple des sels de sodium tel que l'acétate de sodium, ou leur mélange. De préférence, la solution tampon est de préférence choisie parmi les solutions d'acétate de sodium et d'acide, notamment acide acétique, les solutions de glycine et d'acide chlorhydrique, les solutions d'acide éthanoïque et éthanoate de sodium ou les solutions d'acide citrique et phosphate de sodium.

De préférence, la composition réactive (CR1') de l'invention comprend de l'éthanol ou du 1,4-butanediol (de préférence éthanol), de l'acide sulfurique, du chlorhydrate d'hydroxylamine et de l'acétate de sodium en solution dans l'eau.

La troisième composition réactive (CR3) de la méthode selon l'invention comprend au moins un agent déstabilisant d'émulsion. Les première, deuxième et quatrième compositions réactives ainsi que la composition réactive CR1' sont des compositions aqueuses. En mélange avec la composition lubrifiante ces compositions aqueuses peuvent former une émulsion. La troisième composition réactive permet donc, de manière avantageuse, de casser l'émulsion ainsi obtenue et par conséquent de permettre un meilleur déphasage. Ainsi, la troisième composition réactive de la méthode selon l'invention, permet de façon avantageuse de faciliter et d'accélérer le passage des ions ferriques et ferreux de la phase huileuse de la composition lubrifiante vers la phase aqueuse amenée par les première, deuxième et quatrième compositions réactives ou par la composition réactive CR1'. Avantageusement, l'agent déstabilisant est constitué de composé non-miscible à l'eau L'agent déstabilisant de l'invention est de préférence choisi parmi les alcools primaires ou secondaires (C4 à C10), seuls ou en mélange. De préférence, l'agent déstabilisant de l'invention est choisi parmi l'alcool isoamylique, l'octan-1-ol, l'octan-2-ol, le 1-heptanol, 2-ethyl-hexanol, 2-ethyl-butanol, seuls ou en mélange, de préférence l'alcool isoamylique, l'octan-1-ol, l'octan-2-ol, seuls ou en mélange, de préférence l'alcool isoamylique, le 1-heptanol, 2-ethyl-hexanol, 2-ethyl-butanol, seuls ou en mélange, de préférence l'alcool isoamylique.

De préférence, la troisième composition réactive selon la méthode de la présente invention comprend de 20 à 100 % en poids d'agent déstabilisant d'émulsion.

La troisième composition réactive de la présente invention peut en outre comprendre des additifs, par exemple lorsque l'agent déstabilisant présente un point éclair trop important, la troisième composition réactive selon l'invention peut comprendre des co-solvants permettant de diminuer le point éclair de la composition. Parmi ces co-solvants, on peut par exemple citer les distillats légers de pétrole de préférence comprenant moins de 2% de composés aromatiques, tels que par exemples les hydrocarbures, de préférence en C9 à C16, par exemple n-alcane en C9 à C16, isoalcane en C9 à C16, les hydrocarbures cycliques en C9 à C16.

La quatrième composition réactive (CR4) de la méthode selon la présente invention comprend un agent complexant des ions ferreux. Cet agent complexant est choisi parmi les agents complexant les ions ferreux et dont la complexation est à l'origine d'un changement de couleur pouvant être détecté par mesure de l'absorbance de la solution obtenue par un spectrophotomètre. L'absorbance mesurée peut ensuite être reliée à la teneur en ions ferreux, et donc en ions fer, de l'échantillon analysé, exprimée en ppm. De préférence, cet agent complexant est choisi parmi la ferrozine, le ferene, la phénantroline et ses dérivés, par exemple bathophenantroline, la bipyridine, l'acide thioglycolique, le sel R-nitroso (sel sodique de l'acide 3-hydroxy-4-nitroso-2,7-naphtalenedisulfonique), le ferricyanurate de potassium (hexacyanoferrate(III) de potassium), le 2,4,6-tripirydyl-s-triazine (TPTZ). De préférence, l'agent complexant est choisi parmi le TPTZ, le ferricyanurate de potassium ou le sel R-nitroso. De préférence, l'agent complexant est le sel R-nitroso.

De préférence, la quatrième composition réactive comprend de 0,5 à 5 % en poids de l'agent complexant. De préférence, la composition réactive comprend 1% en poids de l'agent complexant.

La méthode de dosage de la présente invention est une méthode de dosage colorimétrique mettant en œuvre une mesure de l'absorbance de la solution obtenue par complexation de l'agent complexant avec les ions ferreux par spectrométrie UV-visible, à l'aide d'un spectrophotomètre, de préférence un spectrophotomètre électronique. Ainsi, les mesures d'absorbance des étapes d) et g) sont réalisées à l'aide d'un spectrophotomètre, de préférence un spectrophotomètre électronique.

La mesure de l'absorbance par le spectrophotomètre est basée sur la loi de Beer Lambert. Une lumière incidente d'intensité I0 traverse la solution aqueuse à analyser, une partie de cette lumière est absorbée par la solution, l'intensité I résultante est transmise à travers la solution. L'absorbance (A) de la solution est alors déterminée par la relation suivante :

$$A = log\left(\frac{I0}{I}\right)$$

Il est possible de relier l'absorbance à la concentration en ions ferreux dans la solution à l'aide d'une courbe d'étalonnage donnant une droite reliant l'absorbance à la concentration en ions ferreux de solution de concentration connue. Le spectrophotomètre électronique est préalablement calibré avec cette courbe d'étalonnage et l'absorbance mesurée permet de déterminer la quantité d'ions ferreux en ppm dans l'échantillon à analyser.

Dans un premier mode de réalisation, une mesure à blanc de l'absorbance peut être réalisée sur un récipient comprenant le mélange des quatre compositions réactives définies ci-dessus. Cette mesure à blanc est alors considérée comme référence dans le spectrophotomètre et chacune des mesures réalisées sur les compositions obtenues à l'étape (f) sera comparée à cette valeur à blanc pour donner la valeur réelle de l'absorbance comme expliqué ci-dessous.

Dans un second mode de réalisation de l'invention, et afin d'obtenir une valeur fiable et juste de l'absorbance et donc indirectement de la concentration des ions ferreux, un blanc est réalisé pour prendre en compte l'influence du second récipient et des première, deuxième, troisième et quatrième compositions réactives sur la mesure de l'absorbance. Pour cela, une étape d) de mesure de l'absorbance est réalisée sur le mélange obtenu à l'étape c) dans le second récipient. Par différence entre l'absorbance obtenue à l'étape g) et celle obtenue à l'étape c) on obtient l'absorbance réelle de la solution obtenue à l'étape f) et on peut ainsi déterminer la quantité correspondante en ions fer. De façon particulièrement avantageuse, le spectrophotomètre électronique est programmé pour faire la différence entre les absorbances mesurées aux étapes d) et g) et ensuite la concentration en ions fer correspondante dans la solution à analyser en ppm.

Lors de l'étape g), le second récipient est placé dans le spectrophotomètre de sorte que l'émission de lumière incidente traverse uniquement la phase aqueuse. Comme cela sera vu par la suite, le spectrophotomètre présente de manière préférée une zone de réception de l'échantillon adaptée, de préférence avec un angle adapté, de façon à ce que l'émission de lumière incidente traverse uniquement la phase aqueuse.

De manière particulièrement avantageuse, l'ensemble de ces étapes d), g) et h) sont mises en œuvre directement par le spectrophotomètre électronique qui est programmé pour cela.

De préférence, l'agitation de l'étape f) est réalisée par retournement du récipient au moins 5 fois, par exemple 10 fois. Après agitation, le récipient est directement introduit dans le spectrophotomètre. De façon avantageuse, l'agitation est une agitation douce.

De préférence, la mesure lors de l'étape g) est réalisée en moins de 10 minutes après introduction du récipient dans le spectrophotomètre, de préférence, la mesure est réalisée 5 minutes après l'introduction du récipient dans le spectrophotomètre, ce temps représentant le temps nécessaire à la réaction de complexation de l'agent complexant avec les ions ferreux.

De préférence, les première, deuxième, troisième et quatrième compositions réactives sont introduites chacune à raison de 5 à 10 ml dans le second récipient. De préférence, les première, deuxième, troisième et quatrième compositions réactives sont introduites chacune à raison de 5 ml dans le second récipient.

De préférence, les troisième et quatrième compositions réactives ainsi que la composition CR1' sont introduites chacune à raison de 5 à 10 ml dans le second récipient. De préférence, les troisième et quatrième compositions réactives ainsi que la composition CR1' sont introduites chacune à raison de 5 ml dans le second récipient.

Dans un autre mode de réalisation, les première, deuxième et troisième compositions réactives peuvent être contenues dans une seule composition réactive (CR) préparée préalablement.

De préférence 1 à 10 gouttes, de préférence 3 gouttes, soit environ 0,075g ou 100 $\mu$l, de composition lubrifiante à analyser sont introduites à l'étape e) dans le second récipient.

La méthode selon la présente invention peut être répétée autant de fois que l'on a d'échantillons à analyser. Dans ce cas, il y aura autant de premier et second récipient que de compositions lubrifiantes à analyser. De manière particulièrement avantageuse, si l'étape d) est mise en œuvre, il conviendra de bien faire les étapes d) et g) pour chaque échantillon à analyser l'une après l'autre et de ne pas faire les étapes d) pour chacun des échantillons à analyser puis les étapes g) pour chacun des échantillons à analyser, en effet, le spectrophotomètre est de préférence paramétré pour comparer chacun des mélanges obtenus aux étapes c) et f).

De façon particulièrement avantageuse, la méthode selon la présente invention est fiable, elle permet de doser de 0 à 900 ppm de fer dans les échantillons, de préférence de 50 à 700 ppm.

De façon avantageuse, la méthode selon l'invention est simple et rapide et ne nécessite pas de connaissances particulières de l'opérateur. De façon avantageuse, la méthode selon l'invention permet d'obtenir un résultat sur la quantité d'ions fer dans une composition lubrifiante usagée en moins de 10 minutes et avec une précision de l'ordre de plus ou moins 10 ppm.

De façon particulièrement avantageuse, la méthode de la présente invention ne nécessite pas le chauffage des échantillons à analyser, ni la digestion du fer particulaire.

La présente invention concerne également un kit selon la revendication 13 pour la mise en œuvre de la méthode décrite ci-dessus. Ce kit comprend :

- une première composition réactive (CR1) aqueuse comprenant au moins un agent d'extraction des ions ferriques et ferreux de la phase huileuse de la composition lubrifiante vers la phase aqueuse ;
- une deuxième composition réactive (CR2) aqueuse comprenant au moins un agent de réduction des ions ferriques (Fe3+) en ions ferreux (Fe2+) ;
- une troisième composition réactive (CR3) comprenant au moins un agent déstabilisant d'émulsion ;
- une quatrième composition réactive (CR4) en solution aqueuse comprenant un agent complexant des ions ferreux, cet agent CR4 changeant de couleur lors de sa complexation avec les ions ferreux ;
- un spectrophotomètre, de préférence un spectrophotomètre électronique ;
- au moins un premier récipient ;
- au moins un second récipient ;

- au moins un dispositif (D1) pour le prélèvement de la composition lubrifiante à analyser ;
- un dispositif (D2) pour chacun des prélèvements des première, deuxième, troisième et quatrième compositions réactives ;
- au moins un dispositif (D3) pour le prélèvement des quelques gouttes de la composition lubrifiante à analyser lors de l'étape e); caractérisé en ce que le kit comprend un support aimanté destiné à recevoir l'au moins un premier récipient.

CR1, CR2, CR3 et CR4 sont telles que définies plus haut.

Le spectrophotomètre, de préférence spectrophotomètre électronique, comprend un dispositif d'émission de lumière, par exemple une diode électroluminescente ; un capteur photoélectrique ; une zone de réception du second récipient ; un logiciel de traitement des données de l'absorbance pour conversion en concentration d'ions ferreux en ppm. De préférence, la zone de réception du second récipient est positionnée entre 10 et 50 mm, de préférence entre 15 et 30 mm, par exemple 24 mm du fond du second récipient et orientée avec un angle de 35 à 75°, de préférence de 50 à 70°, par exemple de 60° pour permettre une mesure de l'absorbance uniquement dans la phase aqueuse du mélange obtenu à l'étape f) dans laquelle se trouve le complexe coloré formé entre l'agent complexant et les ions ferreux.

De préférence, le premier récipient est en matière plastique ou en verre, de préférence en matière plastique. Il comprend éventuellement un bouchon. De préférence, le kit comporte au moins autant de premier récipient que d'échantillons de composition lubrifiante à analyser. En effet, les moteurs comprennent entre 4 et 14 cylindres, il y a donc au minimum entre 4 et 14 échantillons de composition lubrifiante à analyser en même temps (un échantillon de composition lubrifiante par cylindre). De préférence, il y a au moins un premier récipient, de préférence il y a entre un et 50 premiers récipients, par exemple entre 5 et 40 premiers récipients.

De préférence, le second récipient est un récipient sous forme de tube muni d'un bouchon, de préférence un tube à essai muni d'un bouchon. Il peut être en matière plastique ou en verre, de préférence en verre. De préférence, le kit comporte autant de second récipient que d'échantillons de compositions lubrifiantes à analyser. Le kit comprend donc entre 1 et 100 seconds récipients, par exemple entre 10 et 80 seconds récipients, par exemple 50 seconds récipients.

Les premier et second récipients peuvent être des récipients en verre ou en plastique, ils peuvent être jetables ou nettoyables. De préférence, dans le cadre de l'invention les premier et second récipients sont jetables.

De préférence, le dispositif (D1) pour le prélèvement de la composition lubrifiante à analyser est une seringue, de préférence en matière plastique jetable. Le kit peut comprendre autant de dispositif (D1) que de compositions lubrifiantes à analyser, de préférence le kit comprend de 1 à 100 dispositifs (D1), de préférence de 10 à 80, par exemple 50.

De préférence, chacun des dispositifs (D2) sont des seringues, de préférence en matière plastique jetable. Le kit peut comprendre de 3 à 18 dispositifs (D2), de préférence de 3 à 6 dispositifs (D2).

De préférence, le support aimanté est un support destiné à recevoir le premier récipient comprenant la composition lubrifiante à analyser et comprenant un aimant en son fond. De préférence, le support aimanté est un rack comprenant de 1 à 50, de préférence 7 emplacements pour la réception des premiers récipients. De préférence, le support aimanté est en deux parties, une partie inférieure comprenant les emplacements pour la réception des premiers récipients, chacun des emplacements comprenant un aimant, et une partie supérieure comprenant des emplacements pour les seconds récipients, les emplacements de la partie inférieure et les emplacements de la partie supérieure étant de préférence placés l'un en face de l'autre afin d'identifier rapidement et simplement les compositions lubrifiantes à analyser.

De préférence, le dispositif (D3) pour le prélèvement des quelques gouttes de la composition lubrifiante à analyser lors de l'étape e) est une pipette, de préférence en matière plastique jetable ou une micropipette à déplacement positif, par exemple de 100 μl, par exemple de type Microman Gilson ou Transferpettor Brand. Il peut y avoir au moins autant de dispositif (D3) que de compositions lubrifiantes à analyser. Par exemple, le kit peut comprendre de 1 à 50 dispositifs (D3), de préférence 50 dispositifs (D3).

De façon particulièrement avantageuse, le kit selon l'invention peut être rangé dans un coffre ou une valise et est par conséquent facilement transportable.

La figure (1) représente un kit selon l'invention.

La figure (2) représente un support aimanté selon l'invention.

Le kit de la figure (1) comprend :

- un support aimanté (1) comprenant des emplacements (2) comprenant chacun un aimant et destinés à recevoir des premiers récipients (3) et des emplacements (4) destinés à recevoir des seconds récipients (5) ;
- des premiers récipients (3) ;
- des seconds récipients (5) ;
- une composition réactive (CR1') (6) ;
- une composition réactive (CR3) (7) ;
- une composition réactive (CR4) (8) ;

- un spectrophotomètre électronique (9) comprenant un emplacement (10) pour la réception des seconds récipients, de préférence, cet emplacement faisant un angle de 35 à 75°, de préférence de 50 à 70°, par exemple de 60° par rapport au corps du spectrophotomètre ;
- au moins un dispositif (D3) (11) ; et
- au moins trois dispositifs (D2) (12).

La figure (2) représente un support aimanté (1) comprenant des emplacements (2) comprenant chacun un aimant et destinés à recevoir des premiers récipients (3) et des emplacements (4) destinés à recevoir des seconds récipients (5).

La présente invention va maintenant être décrite à l'aide d'exemple non limitatif de mise en œuvre de la méthode et du kit de l'invention.

Exemple

L'opérateur met en route le spectrophotomètre.

a) L'opérateur recueille dans des premiers récipients (3) les compositions lubrifiantes à analyser et place ces récipients dans les emplacements (2) comprenant des aimants du support aimanté (1), ce par quoi le fer particulaire éventuellement présent dans la composition lubrifiante à analyser est attiré vers le bas du récipient à proximité de l'aimant.

b) L'opérateur ajoute ensuite, en utilisant trois dispositifs D2 (12) distincts chacune des compositions réactives CR1' (6), CR3 (7) et CR4 (8), dans autant de second récipient (5) qu'il y a d'huiles à analyser. Le contenu des seconds récipients sont mélangés par retournement des seconds récipients (5). Tous les seconds récipients (5) sont disposés dans les emplacements (4) du support aimanté (1).

c) L'opérateur introduit ensuite l'un des seconds récipients (5) dans l'emplacement (10) du spectrophotomètre (9) et mesure l'absorbance de la solution contenue dans le second récipient (5).

d) Ce second récipient (5) est ensuite replacé dans un des emplacements (4) du support aimanté (1) et l'opérateur y ajoute trois gouttes, à l'aide d'un dispositif D3 (11), d'une des compositions lubrifiantes à analyser contenue dans l'un des premiers récipients (3). Pour ce faire l'opérateur doit bien veiller à ne pas introduire le dispositif D3 au fond du récipient (3) afin de ne pas prélever le fer particulaire éventuellement présent au fond du récipient (3). Après l'ajout de la composition lubrifiante l'opérateur note un changement de couleur résultant de la réaction entre les ions fer et l'agent complexant de la composition réactive (CR4).

e) Le récipient (5) est ensuite mélangé en le retournant 10 fois puis introduit dans l'emplacement (10) du spectrophotomètre (9).

f) Le récipient (5) est laissé 5 minutes dans l'emplacement (10) du spectrophotomètre (9) avant le lancement de la mesure de l'absorbance de la solution contenue dans ledit second récipient (5).

g) Le spectrophotomètre calcule ensuite la différence entre la mesure de l'absorbance lorsque le récipient (5) ne comprend que les compositions réactives CR1', CR3 et CR4 et la mesure de l'absorbance lorsque la composition lubrifiante a été ajoutée dans le récipient (5).

h) A partir de la mesure obtenue en g) le spectrophotomètre détermine la quantité d'ions fer contenus dans la composition lubrifiante à analyser.

Les étapes c) à h) sont ensuite réitérées pour chacune des compositions lubrifiantes à analyser.

**Revendications**

1. Méthode de dosage des ions fer dans des compositions lubrifiantes, comprenant les étapes suivantes :

a) Prélèvement d'un échantillon de la composition lubrifiante à analyser qui est introduit dans un premier récipient ;

b) Placement du premier récipient contenant l'échantillon à analyser sur un aimant ;

c) Ajout dans un second récipient :

  ◦ d'une première composition réactive, CR1 aqueuse comprenant au moins un agent d'extraction des ions ferriques et ferreux d'une phase huileuse d'une composition lubrifiante vers une phase aqueuse, l'eau provenant notamment de la composition CR1

  ◦ d'une deuxième composition réactive, CR2 aqueuse comprenant au moins un agent de réduction des

ions ferriques $Fe^{3+}$ en ions ferreux, $Fe^{2+}$ ;
◦ d'une troisième composition réactive, CR3 comprenant au moins un agent déstabilisant d'émulsion ; et
◦ d'une quatrième composition réactive, CR4 en solution aqueuse comprenant un agent complexant des ions ferreux, **caractérisé en ce que** cet agent change de couleur lors de sa complexation avec les ions ferreux;

et mélange des première, deuxième, troisième et quatrième compositions réactives;
d) optionnellement, mesure photochimique de l'absorbance du mélange obtenu à l'étape c) ;
e) Prélèvement de gouttes sur le surnageant de la composition lubrifiante contenue dans le premier récipient, maintenu en place sur l'aimant, et ajout de ces gouttes dans le second récipient comprenant le mélange des première, deuxième, troisième et quatrième compositions réactives obtenu à l'étape c) ;
f) Agitation du mélange obtenu à l'étape e) ;
g) Mesure photochimique de l'absorbance du mélange obtenu à l'étape f) ;
h) Détermination de la quantité d'ions ferreux dans l'échantillon de la composition lubrifiante à partir des mesures obtenues à l'étape g) ou par différence entre l'absorbance obtenue à l'étape g) et celle obtenue à l'étape d) si cette dernière est mesurée.

2. Méthode selon la revendication 1, dans laquelle l'au moins un agent d'extraction est choisi parmi des agents solubilisant les ions ferriques et ferreux et étant immiscibles à la composition lubrifiante.

3. Méthode selon la revendication 1 ou 2, dans laquelle l'au moins un agent d'extraction est choisi parmi acide sulfurique, acide nitrique, acide hydrochlorhydrique, acide phosphorique, seuls ou en mélange.

4. Méthode selon l'une des revendications 1 à 3, dans laquelle l'au moins un agent d'extraction est l'acide sulfurique.

5. Méthode selon l'une quelconque des revendications 1 à 4, dans laquelle l'au moins un agent de réduction des ions ferriques en ions ferreux est choisi parmi hydroquinone, chlorhydrate d'hydroxylamine, hydrazine, dithionite, seuls ou en mélange.

6. Méthode selon la revendication 5, dans laquelle l'au moins un agent de réduction des ions ferriques en ions ferreux est le chlorhydrate d'hydroxylamine.

7. Méthode selon l'une quelconque des revendications 1 à 6, dans laquelle l'au moins un agent déstabilisant est choisi parmi des alcools primaires ou secondaires possédant 4 à 10 atomes de carbone non miscibles à l'eau, seuls ou en mélange.

8. Méthode selon la revendication 7, dans laquelle l'au moins un agent déstabilisant est choisi parmi l'alcool isoamylique, octan-1-ol, octan-2-ol, le 1-heptanol, le 2-ethyl-hexanol, et le 2-ethyl-butanol.

9. Méthode selon l'une quelconque des revendications 1 à 8, dans laquelle l'agent complexant est choisi parmi des agents complexant les ions ferreux et dont la complexation est à l'origine d'un changement de couleur pouvant être détecté par mesure de l'absorbance de la solution obtenue à l'étape e) par un spectrophotomètre.

10. Méthode selon l'une quelconque des revendications 1 à 9, dans laquelle l'agent complexant est choisi parmi la ferrozine, le ferene, la phénantroline et ses dérivés, la bipyridine, l'acide thioglycolique, le sel R-nitroso (sel sodique de l'acide 3-hydroxy-4-nitroso-2,7-naphtalenedisulfonique), le ferricyanurate de potassium (hexacyanoferrate(III) de potassium), et le 2,4,6-tripirydyl-s-triazine (TPTZ).

11. Méthode selon la revendication 9 ou 10, dans laquelle l'agent complexant est le sel R-nitroso.

12. Méthode selon l'une quelconque des revendications 1 à 11, dans laquelle les première et deuxième composition réactive sont comprises dans une composition réactive aqueuse CR1'.

13. kit pour la mise en œuvre de la méthode selon l'une quelconque des revendications 1 à 12 comprenant :

- une première composition réactive, CR1 (6) aqueuse comprenant au moins un agent d'extraction des ions ferriques et ferreux de la phase huileuse de la composition lubrifiante vers la phase aqueuse tel que défini aux revendications 1 à 4 ;

- une deuxième composition réactive, CR2 (6) aqueuse comprenant au moins un agent de réduction des ions ferriques, $Fe^{3+}$ en ions ferreux, $Fe^{2+}$ tel que défini aux revendications 1, 5 et 6 ;
- une troisième composition réactive, CR3 (7) comprenant au moins un agent déstabilisant d'émulsion tel que défini aux revendications 1, 7 et 8 ;
- une quatrième composition réactive, CR4 (8) en solution aqueuse comprenant un agent complexant des ions ferreux, tel que défini aux revendications 1, 9 à 11, cet agent CR4 changeant de couleur lors de sa complexation avec les ions ferreux ;
- un spectrophotomètre, de préférence un spectrophotomètre électronique (9) ;
- au moins un premier récipient (3) ;
- au moins un second récipient (5) ;
- au moins un dispositif, D1 pour le prélèvement de la composition lubrifiante à analyser ;
- un dispositif, D2 (12) pour chacun des prélèvements des première, deuxième, troisième et quatrième compositions réactives ;
- au moins un dispositif, D3 (11) pour le prélèvement des gouttes de la composition lubrifiante à analyser lors de l'étape e);

**caractérisé en ce que** le kit comprend un support aimanté (1) destiné à recevoir l'au moins un premier récipient (3).

## Patentansprüche

1. - Verfahren zum Bestimmen von Eisenionen in Schmierstoffzusammensetzungen, das Verfahren umfassend die folgenden Schritte:

   a) Entnehmen einer Probe der zu analysierenden Schmierstoffzusammensetzung, die in einen ersten Behälter gefüllt wird;
   b) Platzieren des ersten Behälters, der die zu analysierende Probe enthält, auf einem Magneten;
   c) Hinzufügen in einen zweiten Behälter:

   ◦ einer ersten reaktiven wässrigen Zusammensetzung, CR1, umfassend mindestens ein Mittel zum Extrahieren von Eisen(III)- und Eisen(II)-Ionen F aus einer öligen Phase einer Schmierstoffzusammensetzung in eine wässrige Phase, wobei das Wasser insbesondere aus der Zusammensetzung CR1 stammt
   ◦ einer zweiten reaktiven wässrigen Zusammensetzung, CR2, umfassend mindestens ein Reduktionsmittel von Eisen(III)-Ionen $Fe^{3+}$ zu Eisen(II)-Ionen $Fe^{2+}$,
   ◦ einer dritten reaktiven Zusammensetzung, CR3, umfassend mindestens ein emulsionsdestabilisierendes Mittel; und
   ◦ einer vierten reaktiven Zusammensetzung, CR4, in wässriger Lösung, umfassend ein Mittel zum Komplexieren von Eisen(II)-Ionen, **dadurch gekennzeichnet, dass** das Mittel bei Komplexierung mit den Eisen(II)-Ionen die Farbe ändert; und Mischen der ersten, der zweiten, der dritten und der vierten reaktiven Zusammensetzung;

   d) optional photochemisches Messen der Absorbanz des in Schritt c) erlangten Gemischs;
   e) Entnehmen von Tropfen aus dem Überstand der Schmierstoffzusammensetzung, die in dem ersten Behälter enthalten ist, der platziert auf dem Magneten gehalten wird, und Hinzufügen dieser Tropfen in den zweiten Behälter, der das in Schritt c) erlangte Gemisch der ersten, der zweiten, der dritten und der vierten reaktiven Zusammensetzung umfasst;
   f) Schütteln des in Schritt e) erlangten Gemischs;
   g) photochemisches Messen der Absorbanz des in Schritt f) erlangten Gemischs;
   h) Bestimmen der Menge an Eisen(II)-Ionen in der Probe der Schmierstoffzusammensetzung aus den in Schritt g) erlangten Messungen oder durch Differenzbildung zwischen der Absorbanz,

   die in Schritt g) erlangt wird, und der, die in Schritt d) erlangt wird, wenn letztere gemessen wird.

2. - Verfahren nach Anspruch 1, wobei das mindestens eine Extraktionsmittel ausgewählt ist aus Mitteln, die Eisen(III)- und Eisen(II)-Ionen solubilisieren und mit der Schmierstoffzusammensetzung unmischbar sind.

3. - Verfahren nach Anspruch 1 oder 2, wobei das mindestens eine Extraktionsmittel ausgewählt ist aus Schwefelsäure, Salpetersäure, Salzsäure, Phosphorsäure, einzeln oder in Gemisch.

**4.** - Verfahren nach einem der Ansprüche 1 bis 3, wobei das mindestens eine Extraktionsmittel Schwefelsäure ist.

**5.** - Verfahren nach einem der Ansprüche 1 bis 4, wobei das mindestens eine Reduktionsmittel von Eisen(III)-Ionen zu Eisen(II)-Ionen ausgewählt ist aus Hydrochinon, Hydroxylaminhydrochlorid, Hydrazin, Dithionit, einzeln oder in Gemisch.

**6.** - Verfahren nach Anspruch 5, wobei das mindestens eine Reduktionsmittel von Eisen(III)-Ionen zu Eisen(II)-Ionen Hydroxylaminhydrochlorid ist.

**7.** - Verfahren nach einem der Ansprüche 1 bis 6, wobei das mindestens eine destabilisierende Mittel ausgewählt ist aus primären oder sekundären Alkoholen mit 4 bis 10 Kohlenstoffatomen, die nicht mit Wasser mischbar sind, allein oder in Gemisch.

**8.** - Verfahren nach Anspruch 7, wobei das mindestens eine destabilisierende Mittel ausgewählt ist aus Isoamylalkohol, Octan-1-ol, Octan-2-ol, 1-Heptanol, 2-Ethylhexanol und 2-Ethylbutanol.

**9.** - Verfahren nach einem der Ansprüche 1 bis 8, wobei der Komplexbildner ausgewählt ist aus Mitteln, die Eisen(II)-Ionen komplexieren und deren Komplexierung eine Farbänderung verursacht, die durch Messen der Absorbanz der in Schritt e) erlangten Lösung mit einem Spektralphotometer festgestellt werden kann.

**10.** - Verfahren nach einem der Ansprüche 1 bis 9, wobei der Komplexbildner ausgewählt ist aus Ferrozin, Feren, Phenantrolin und seinen Derivaten, Bipyridin, Thioglycolsäure, R-Nitroso-Salz (Natriumsalz von 3-Hydroxy-4-nitroso-2,7-naphthalindisulfonsäure), Kaliumferricyanurat (Kaliumhexacyanoferrat(III)) und 2,4,6-Tripirydyl-s-triazin (TPTZ).

**11.** - Verfahren nach Anspruch 9 oder 10, wobei der Komplexbildner das Nitroso-R-Salz ist.

**12.** Verfahren nach einem der Ansprüche 1 bis 11, wobei die erste und die zweite reaktive Zusammensetzung in einer wässrigen reaktiven Zusammensetzung CR1' enthalten sind.

**13.** Kit zur Durchführung des Verfahrens nach einem der Ansprüche 1 bis 12, umfassend:

- eine erste wässrige reaktive Zusammensetzung, CR1 (6), umfassend mindestens ein Extraktionsmittel von Eisen(III)- und Eisen(II)-Ionen aus der Ölphase der Schmierstoffzusammensetzung in die wässrige Phase, wie es in den Ansprüchen 1 bis 4 definiert ist;
- eine zweite wässrige reaktive Zusammensetzung, CR2 (6), umfassend mindestens ein Reduktionsmittel von Eisen(III)-Ionen, Fe$^{3+}$ zu Eisen(II)-Ionen, Fe$^{2+}$, wie es in den Ansprüchen 1, 5 und 6 definiert ist;
- eine dritte reaktive Zusammensetzung, CR3 (7), umfassend mindestens ein emulsionsdestabilisierendes Mittel, wie es in den Ansprüchen 1, 7 und 8 definiert ist;
- eine vierte reaktive Zusammensetzung, CR4 (8) in wässriger Lösung, umfassend ein Mittel zum Komplexieren von Eisen(II)-Ionen, wie es in den Ansprüchen 1, 9 bis 11 definiert ist, wobei das CR4-Mittel bei seiner Komplexierung mit Eisen(II)-Ionen seine Farbe ändert;
- ein Spektralphotometer, vorzugsweise ein elektronisches Spektralphotometer (9)
- mindestens einen ersten Behälter (3)
- mindestens einen zweiten Behälter (5)
- mindestens eine Vorrichtung, D1 zum Entnehmen der zu analysierenden Schmierstoffzusammensetzung;
- eine Vorrichtung, D2 (12) für jede Entnahme der ersten, der zweiten, der dritten und der vierten reaktiven Zusammensetzungen;
- mindestens eine Vorrichtung, D3 (11) zum Entnehmen von Tropfen der zu analysierenden Schmierstoffzusammensetzung in Schritt e); **dadurch gekennzeichnet, dass** das Kit einen magnetischen Träger (1) umfasst, der dazu bestimmt ist, mindestens einen ersten Behälter (3) aufzunehmen.

**Claims**

**1.** Method for determining iron ions in lubricating compositions, comprising the following steps:

a) Taking a sample of the lubricating compositing to be analysed that is introduced in a first container;

b) Placing said first container containing the sample to be analysed on a magnet;

c) Adding to a second container:

- a first aqueous reactive composition CR1 comprising at least one extracting agent of ferric and ferrous ions from the oil phase of the lubricating composition to an aqueous phase, the water coming from the composition CR1;
- a second aqueous reactive composition CR2 comprising at least one reducing agent of ferric ions $Fe^{3+}$ to ferrous ions $Fe^{2+}$;
- a third reactive composition CR3 comprising at least one emulsion destabilising agent; and
- a fourth reactive composition CR4 in an aqueous solution comprising a complexing agent of ferrous ions, **characterized in that** this agent changes colour when complexing with ferrous ions;

and mixing thereof of the first, second, third and fourth reactive compositions;

d) optionally, photochemical measurement of the absorbance of the mixture obtained at step c);

e) Taking drops of the supernatant of the lubricating composition contained in the first container, held in position on the magnet, and adding drops to the second container comprising the mixture of the first, second, third and fourth reactive compositions obtained at step c);

f) Agitating the mixture obtained at step e);

g) Conducting photochemical measurement of the absorbance of the mixture obtained at step f);

h) Determining the quantity of ferrous ions in the sample of the lubricating composition from the measurements obtained at step g) or from the difference between absorbance obtained at step g) and the one obtained at step d) if this last one is measured.

2. The method according to claim 1, wherein the at least one extracting agent is selected from among agents solubilising ferric and ferrous ions and being immiscible in the lubricating composition.

3. Method according to claim 1 or 2, wherein the at least extracting agent is selected from among sulfuric acid, nitric acid, hydrochloric acid, phosphoric acid, alone or in a mixture.

4. The method according to any one of claims 1 to 3, wherein the at least one extracting agent is sulfuric acid.

5. The method according to any of claims 1 to 4, wherein the at least one reducing agent of ferric ions to ferrous ions is selected from among hydroquinone, hydroxylamine hydrochloride, hydrazine, dithionite, alone or in a mixture.

6. The method according to claim 5, wherein the at least one reducing agent of ferric ions to ferrous ions is hydroxylamine hydrochloride.

7. The method according to any of claims 1 to 6, wherein the at least one destabilising agent is selected from among primary or secondary alcohols comprising from 4 to 10 carbon atoms non-miscible in water, alone or in a mixture.

8. The method according to claim 7, wherein the at least one destabilising agent is isoamyl alcohol, octan-1-ol, octan-2-ol, 1-heptanol, 2-ethyl-hexanol, 2-ethyl-butanol.

9. The method according to any of claims 1 to 8, wherein the complexing agent is selected from among ferrous ion complexing agents and for which complexing is the cause of a change of colour able to be detected by spectrophotometric measurement of the absorbance of the solution obtained at step e) by a spectrophotometer.

10. The method according to any of claims 1 to 9, wherein the complexing agent is selected from among ferrozine, ferene, phenantroline and derivatives thereof e.g. bathophenantroline, bipyridine, thioglycolic acid, nitroso-R salt (sodium salt of 3-hydroxy-4-nitroso-2,7-naphthalenedisulfonic acid), potassium ferricyanide (potassium hexacyanoferrate(III)) and 2,4,6-tripirydyl-s-triazine (TPTZ).

11. The method according to claim 9 or 10, wherein the complexing agent is nitroso-R salt.

12. The method according to any of claims 1 to 11, wherein the first and second reactive compositions are included in an aqueous reactive composition CR1'.

13. Kit to implement the method according to any of claims 1 to 12, comprising:

- a first aqueous reactive composition CR1 (6) comprising at least one extracting agent of ferric and ferrous ions from the oil phase of the lubricating composition towards the aqueous phase such as defined in claims 1 to 4;
- a second aqueous reactive composition CR2 (6) comprising at least one reducing agent of ferric ions $Fe^{3+}$ to ferrous ions $Fe^{2+}$ such as defined in claims 1, 5 and 6;
- a third reactive composition CR3 (7) comprising at least one emulsion destabilising agent such as defined in claims 1, 7 and 8;
- a fourth reactive composition CR4 (8) in an aqueous solution comprising a ferrous ion complexing agent such as defined in claims 1, 9 and 11, **characterized in that** this agent CR4 changes colour when complexing with ferrous ions;
- a spectrophotometer, preferably an electronic spectrophotometer (9);
- at least one first container (3);
- at least one second container (5);
- at least one device D1 to take a sample of lubricating composition to be analysed;
- a device D2 (12) to take up each of the first, second, third and fourth reactive compositions;
- at least one device D3 (11) to take up drops of lubricating composition to be analysed at step e),

wherein the kit comprises a magnetised support (1) intended to receive the at least one first container (3).

EP 3 245 511 B1

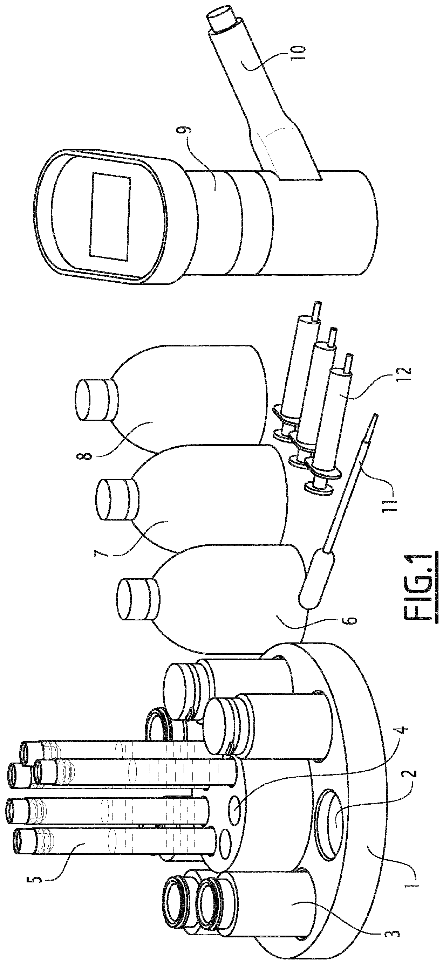

FIG.1

15

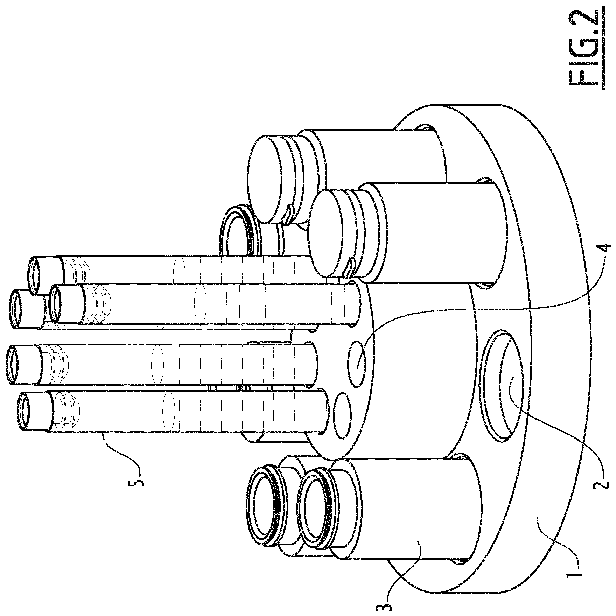

FIG.2

## RÉFÉRENCES CITÉES DANS LA DESCRIPTION

**Documents brevets cités dans la description**

- WO 2006127098 A
- US 4238197 A